# EUROPÄISCHE PATENTSCHRIFT

(11) **EP 0 665 228 B1**
(45) Veröffentlichungstag und Bekanntmachung des Hinweises auf die Patenterteilung: **14.07.1999**
(21) Anmeldenummer: 95100954.7
(22) Anmeldetag: 25.01.1995
(51) Int. Cl.: C07D 471/08, C07D 471/10, A61K 31/435

(54) **Neue 3-Phenylsulfonyl-3,7-diazabicyclo(3,3,1)nonan-Verbindungen enthaltende Arzneimittel**
Pharmaceutical compositions containing 3-phenylsulfonyl-3,7-diazabicyclo 3,3,1 nonanes
Compositions pharmaceutiques contenant 3-phenylsulfonyl-3,7-diazabicyclo 3,3,1 nonanes

(30) Priorität: 01.02.1994 DE 4402931
(43) Veröffentlichungstag der Anmeldung: 02.08.1995
(73) Patentinhaber: Solvay Pharmaceuticals GmbH, 30173 Hannover (DE)
(72) Erfinder: Schön, Uwe, Dr., D-31303 Burgdorf (DE); Farjam, Arman, Dr., D-41179 Mönchengladbach (DE); Brückner, Reinhard, Dr., D-30559 Hannover (DE); Ziegler, Dieter, Dr., D-30966 Hemmingen (DE)
(74) Vertreter: Lauer, Dieter, Dr.

(56) Entgegenhaltungen:
- WO-A-91/07405
- DE-A- 3 722 134
- CHEMICAL ABSTRACTS, vol. 113, no. 13, 1990 Columbus, Ohio, US; abstract no. 115277q, S.A. ZISMAN ET AL. 'The preparation of amide derivatives of 3-azabicyclo[3.3.1]nonanes as new potential antiarrhythmic agents' Seite 692; & ORG. PREP. PROCED. INT., Bd. 22,Nr. 2, 1990 Seiten 255-264,

## Beschreibung

Die vorliegende Erfindung betrifft die Verwendung von 3,7,9,9-tetrasubstituierten 3,7-Diazabicyclo[3,3,1]nonan-Verbindungen, welche in 3-Stellung einen im Phenylring substituierten Phenylsulfonylrest tragen, und deren Salzen als antiarrhythmisch wirksame pharmakologische Wirkstoffe und diese Verbindungen enthaltende pharmazeutische Zubereitungen sowie neue 3,7,9,9-tetrasubstituierte 3-Phenylsulfonyl-3,7-diazabicyclo[3,3,1]nonan-Verbindungen mit antiarrhytbmischen Eigenschaften und Verfahren und Zwischenprodukte zur Herstellung dieser Verbindungen.

Aus der DE-OS 37 22 134 sind 3-Sulfonyl-3,7-diazabicyclo[3,3,1]nonan-Derivate bekannt, welche im gastrointestinalen Trakt eine die Motilität des Magens regulierende Wirkung ausüben.

Aus der WO-A-91/07405 und Chem. Abs. 113, 1152779 (1990) sind 3-Azabicyclo[3.3.1]nonan-Derivate bekannt, welche eine antiarrythmische Wirkung ausüben.

Der Erfindung liegt die Aufgabe zugrunde, neue antiarrhythmisch wirksame Wirkstoffe und pharmazeutische Zubereitungen mit verbessertem Wirkungsprofil zu entwickeln. Ferner liegt der Erfindung die Aufgabe zugrunde, neue 3,7-Diazabicyclo[3,3,1]nonan-Verbindungen mit wertvollen pharmakologischen Eigenschaften zu entwickeln.

Es wurde nun gefunden, daß eine Gruppe von in 3-Stellung durch einen substituierten Phenylsulfonylrest substituierten 3,7-Diazabicyclo[3,3,1]nonan-Verbindungen wertvolle pharmakologische Eigenschaften zur Behandlung von Herzrhythmusstörungen besitzen und ein antiarrhythmisches Wirkprofil zeigen, welches sie zur Behandlung von Herzrhythmusstörungen, insbesondere tachycarden Arrhythmien geeignet macht.

Erfindungsgemäß werden als antiarrhythmische Wirkstoffe zur Herstellung von antiarrhythmisch wirksamen pharmazeutischen Zubereitungen zur Behandlung von Herzrhythmusstörungen in größeren Säugetieren und Menschen 3-Phenylsulfonyl-3,7-diazabicyclo[3,3,1]nonan-Verbindungen der allgemeinen Formel I
(s. Formel I)
worin
- R¹: eine Alkylgruppe mit 1-6 Kohlenstoffatomen oder eine Cycloalkylalkylgruppe mit 4-7 Kohlenstoffatomen bedeutet,
- R²: C₁-C₄ Alkyl bedeutet und
- R³: C₁-C₄ Alkyl bedeutet oder
- R² und R³: gemeinsam eine Alkylenkette mit 3-6 Kohlenstoffatomen bilden,
- R⁴: für Halogen, Nitro, Trifluormethyl oder Cyano, für eine R⁶-O-CO-Gruppe, worin R⁶ C₁-C₄ Alkyl bedeutet, für eine R⁷-SO₂-NH-Gruppe, worin R⁷ C₁-C₄ Alkyl bedeutet, oder für eine R⁸-CO-NH-Gruppe, worin R⁸ C₁-C₄ Alkyl oder eine Phenylgruppe, welche gegebenenfalls durch Halogen, Cyano, Nitro oder einen R⁹-SO₂-Rest, worin R⁹ C₁-C₄ Alkyl ist, substituiert ist, bedeutet, oder für einen in 4-Stellung des Phenylringes angeordneten Imidazolylrest steht, und
- R⁵: Wasserstoff oder Halogen bedeutet, und
deren physiologisch verträgliche Säureadditionssalze verwendet.

Sofern in den Verbindungen der Formel I R¹ für eine Alkylgruppe steht, kann diese geradkettig oder verzweigt sein und 1 bis 6, vorzugsweise 3 bis 5, insbesondere 4 Kohlenstoffatome enthalten. Eine Cycloalkylalkylgruppe R¹ kann 4 bis 9, vorzugsweise 4 bis 7, Kohlenstoffatome enthalten. Als besonders geeignete Reste R¹ haben sich Alkylreste mit 3 bis 5 Kohlenstoffatomen erwiesen.

Sofern die Substituenten R² und R³ C₁-C₄ Alkyl darstellen, können diese Alkylgruppen geradkettig oder verzweigt sein und 1 bis 4, vorzugsweise 1 bis 3 Kohlenstoffatome enthalten und insbesondere Methyl bedeuten. Sofern R² und R³ gemeinsam eine Alkylengruppe bilden, kann diese 3 bis 6, vorzugsweise 4 bis 5, Kohlenstoffatome enthalten. Insbesondere haben sich solche Verbindungen, worin R² und R³ je C₁-C₄ Alkyl, insbesondere Methyl, bedeuten, als geeignet erwiesen.

Sofern der Substituent R⁴ eine C₁-C₄ Alkylgruppe enthält, kann diese geradkettig oder verzweigt sein und 1 bis 4, insbesondere 1 bis 3, Kohlenstoffatome enthalten und vorzugsweise die Methylgruppe darstellen. Vorzugsweise steht der Substituent R⁴ für Cyano oder für einen in 4-Stellung des Phenylringes angeordneten Imidazolylrest.

Als pharmakologisch annehmbare Säureadditionssalze der Verbindungen der Formel I eignen sich beispielsweise deren Salze mit anorganischen Säuren, z. B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Malonsäure, Maleinsäure, Fumarsäure, Milchsäure, Weinsäure, Essigsäure oder Zitronensäure, oder mit organischen Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure.

Gegenstand der Erfindung sind die Verwendung der Verbindungen der Formel I als antiarrhythmisch wirksame pharmakologische Wirkstoffe zur Herstellung von Arzneimitteln und unter Verwendung dieser Wirkstoffe hergestellte antiarrhythmisch wirksame Arzneimittel.

3-Phenylsulfonyl-3,7-diazabicyclo[3,3,1]nonan-Verbindungen der allgemeinen Formel Ia
(s. Formel Ia)
worin
- R¹: eine Alkylgruppe mit 1-6 Kohlenstoffatomen oder eine Cycloalkylalkylgruppe mit 4-7 Kohlenstoffatomen bedeutet,
- R²: C₁-C₄ Alkyl bedeutet und
- R³: C₁-C₄ Alkyl bedeutet oder
- R² und R³: gemeinsam eine Alkylenkette mit 3-6 Kohlenstoffatomen bilden,
- R^{4'}: für Cyano, für eine R⁶-O-CO-Gruppe, worin R⁶ C₁-C₄ Alkyl bedeutet, für eine R⁷-SO₂-NH-Gruppe, worin R⁷ C₁-C₄ Alkyl bedeutet, oder für eine R⁸-CO-NH-Gruppe, worin R⁸ C₁-C₄ Alkyl oder eine Phenylgruppe, welche gegebenenfalls durch Halogen, Cyano, Nitro oder einen R⁹-SO₂-Rest, worin R⁹ C₁-C₄ Alkyl ist, substituiert ist, bedeutet, oder für einen in 4-Stellung des Phenylringes angeordneten Imidazolylrest steht, und
- R⁵: Wasserstoff oder Halogen bedeutet, und
deren physiologisch verträgliche Säureadditionssalze sind bisher in der Literatur noch nicht beschrieben worden und stellen neue wertvolle pharmakologische Wirkstoffe dar, welche ebenfalls Gegenstand der vorliegenden Erfindung sind.

Die übrigen erfindungsgemäß als antiarrhythmische Wirkstoffe verwendeten Verbindungen der Formel I fallen unter den Umfang der in der vorgenannten DE-OS 37 22 134 beschriebenen Verbindungen und sind aus dieser bekannt.

Erfindungsgemäß werden die 3-Phenylsulfonyl-3,7-diazabicyclo[3,3,1]nonan-Verbindungen der Formel I und deren Säureadditionssalze erhalten, indem man in an sich bekannter Weise
a) zur Herstellung von Verbindungen der allgemeinen Formel Ie
   (siehe Formel Ie)
   worin R¹, R², R³ und R⁵ obige Bedeutung besitzen und R^{4'''} für Halogen, Nitro, Trifluormethyl oder Cyano, für eine R⁶-O-CO-Gruppe, worin R⁶ obige Bedeutung besitzt, für eine R⁷-SO₂-NH-Gruppe, worin R⁷ obige Bedeutung besitzt, oder für eine
   R^{8'}-CO-NH-Gruppe, worin R^{8'} C₁-C₄ Alkyl bedeutet, steht
   Verbindungen der allgemeinen Formel II
   (siehe Formel II)
   worin R¹, R² und R³ obige Bedeutung besitzen, mit Verbindungen der allgemeinen Formel III
   (siehe Formel III)
   worin R^{4'''} und R⁵ obige Bedeutung besitzen und Y für eine reaktive Gruppe steht, umsetzt, oder
b) zur Herstellung von Verbindungen der allgemeinen Formel Ic
   (siehe Formel Ic)
   worin R¹, R², R³ und R⁵ obige Bedeutung besitzen, Verbindungen der allgemeinen Formel IV
   (siehe Formel IV)
   worin R¹, R², R³ und R⁵ obige Bedeutung besitzen, mit Imidazol der Formel V
   (siehe Formel V)
   umsetzt, oder
c) zur Herstellung von Verbindungen der allgemeinen Formel Id
   (siehe Formel Id)
   worin R¹, R², R³ und R⁵ obige Bedeutung besitzen und R^{8''} eine Phenylgruppe, welche gegebenenfalls durch Halogen, Cyano, Nitro oder einen R⁹-SO₂-Rest, worin R⁹ C₁-C₄ Alkyl ist, substituiert ist, bedeutet, Verbindungen der allgemeinen Formel VI
   (siehe Formel VI)
   worin R¹, R², R³ und R⁵ obige Bedeutung besitzen, mit Säuren oder reaktionsfähigen Säurederivaten der allgemeinen Formel VII
   (siehe Formel VII)
   worin R^{8''} obige Bedeutung besitzt und X Hydroxy oder eine reaktive Gruppe bedeutet, acyliert,
und gegebenenfalls freie Verbindungen der Formel I in ihre Säureadditionssalze überführt oder die Säureadditionssalze in die freien Verbindungen der Formel I überführt.

Die Umsetzung der Sulfonsäurederivate der Formel III mit den 3,7-Diazabicyclo[3,3,1]nonan-Verbindungen der Formel II gemäß Verfahrensvariante a) kann nach an sich zur Sulfonamidbildung durch Acylierung üblichen Methoden ausgeführt werden. Als reaktionsfähige Derivate kommen insbesondere Sulfonsäurehalogenide, vorzugsweise -chloride, und Anhydride der Sulfonsäuren der Formel IIIb
(s. Formel IIIb)
worin R^{4'''} und R⁵ obige Bedeutung besitzen, in Frage. So kann die reaktive Gruppe Y in den Verbindungen der Formel III beispielsweise Halogen, insbesondere Chlor, oder eine im Phenylring durch R^{4'''} und R⁵ substituierte Phenylsulfonyloxygruppe bedeuten. Die Acylierung kann in einem unter den Reaktionsbedingungen inerten Lösungsmittel bei Temperaturen zwischen 0 °C und Siedetemperatur des Lösungsmittels erfolgen. Als Lösungsmittel eignen sich halogenierte Kohlenwasserstoffe wie Dichlormethan oder Chloroform, aromatische Kohlenwasserstoffe wie Benzol, Toluol oder Chlorbenzol, cyclische Äther wie Tetrahydrofuran oder Dioxan, Dimethylformamid oder Gemische dieser Lösungsmittel. Die Acylierung kann gewünschtenfalls in Gegenwart eines säurebindenden Mittels durchgeführt werden. Als säurebindende Mittel eignen sich anorganische Basen, insbesondere Alkalimetallcarbonate, oder organische Basen, insbesondere tertiäre Niederalkylamine und Pyridine, wie z. B. Triäthylamin oder 4-Dimethylaminopyridin.

Die Umsetzung von Verbindungen der Formel IV mit Imidazol gemäß Verfahrensvariante b) kann auf an sich bekannte Weise in einem unter den Reaktionsbedingungen inerten organischen Lösungsmittel bei erhöhten Temperaturen, beispielsweise Temperaturen zwischen 70 und 150 °C, erfolgen. Als Lösungsmittel eignen sich insbesondere Dimethylsulfoxid, Dimethylformamid und Acetonitril. Zwecymäßigerweise werden Verbindungen der Formel IV mit ca. 3 bis 6 Äquivalenten Imidazol umgesetzt. Im allgemeinen ist es vorteilhaft, unter Zusatz von 1 - 3 Äquivalenten einer anorganischen Base, beispielsweise einem Alkalimetallcarbonat, zu arbeiten.

Die Umsetzung der Amino-Verbindungen der Formel VI mit den Säuren oder Säurederivaten der Formel VII gemäß Verfahrensvariante c) kann nach an sich zur Acylierung von Anilin-Derivaten üblichen Methoden durchgeführt werden. Als reaktionsfähige Säurederivate der Formel III kommen insbesondere Säurehalogenide, vorzugsweise -chloride, und Säureanhydride in Frage. Die Umsetzung derartiger Säurederivate mit den Verbindungen der Formel VI kann unter zur Amidbildung üblichen Reaktionsbedingungen, beispielsweise unter den vorstehend für die Verfahrensvariante a) angegebenen Reaktionsbedingungen durchgeführt werden. Sofern Säuren der Formel VII eingesetzt werden, kann die Umsetzung zweckmäßig in Gegenwart eines aus der Peptidchemie als zur Amidbildung geeignet bekannten Kopplungsreagenzes durchgeführt werden. Als Beispiele von Kopplungsreagentien, welche die Amidbildung mit den freien Säuren dadurch fördern, daß sie mit der Säure in situ unter Bildung eines reaktionsfähigen Säurederivates reagieren, seien insbesondere genannt Alkylcarbodiimide, Carbonyldiimidazol und N-Niederalkyl-2-halogenpyridiniumsalze wie N-Methyl-2-chlorpyridiniumjodid. Die Umsetzung in Gegenwart eines Kopplungsreagenzes kann zweckmäßig in einem inerten organischen Lösungsmittel, beispielsweise einem halogenierten Kohlenwasserstoff und/oder einem aromatischen Kohlenwasserstoff, gegebenenfalls in Gegenwart eines säurebindenden Amins durchgeführt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch vierträgliche Säureadditionssalze überführt werden.

Falls in den Verbindungen der Formel I die Substituenten R² und R³ verschieden sind, enthalten die Verbindungen ein Chiralitätszentrum und können in zwei optisch aktiven Formen oder als Racemat vorliegen. Die vorliegende Erfindung umfaßt sowohl die racemischen Gemische wie auch die optischen Isomeren dieser Verbindungen der Formel I. Die optisch aktiven Verbindungen können aus den racemischen Gemischen in an sich bekannter Weise durch übliche Trennverfahren erhalten werden, z. B. durch chromatographische Trennung an chiralen Trennmaterialien oder fraktionierte Kristallisation geeigneter Salze mit optisch aktiven Säuren. Enantiomerenreine Verbindungen können auch durch Synthese aus entsprechenden enantiomerenreinen Ausgangsverbindungen der Formel II hergestellt werden.

Die Ausgangsbindungen der Formel II sind aus der DE-OS 37 22 134 bekannt und/oder können nach den in dieser DE-OS beschriebenen Methoden oder analog zu den in dieser Schrift beschriebenen Methoden auf an sich bekannte Weise hergestellt werden.

Die Ausgangsverbindungen der Formel III sind bekannt oder können auf an sich bekannte Weise hergestellt werden. Beispielsweise können Aromaten der allgemeinen Formel IX
(siehe Formel IX)
worin R^{4'''} und R⁵ obige Bedeutung besitzen, auf bekannte Weise durch Chlorsulfonsäure und/oder Sulfonylchlorid, in entsprechende substituierte Benzolsulfochloride der Formel III überführt werden.

Die Ausgangsverbindungen der Formel IV können erhalten werden, indem man Verbindungen der Formel II mit Sulfonsäurederivaten der allgemeinen Formel VIII
(s. Formel VIII)
worin Y und R⁵ obige Bedeutung besitzen, umsetzt. Die Umsetzung kann unter den vorstehend für die Umsetzung für Verbindungen der Formel II mit Verbindungen der Formel III angegebenen Bedingungen erfolgen.

Verbindungen der Formel VI sind neue Verbindungen, welche wertvolle Zwischenprodukte zur Herstellung von pharmakologisch wirksamen Verbindungen, beispielsweise Verbindungen der Formel Id, darstellen. Die Verbindungen der Formel VI können erhalten werden, indem man aus entsprechenden Verbindungen der Formel I, worin R⁴ für einen Acylaminorest R^{8'}-CO-NH-, worin R^{8'} obige Bedeutung besitzt, die Acylgruppe R^{8'}-CO-hydrolytisch abspaltet. Die Hydrolyse kann in an sich bekannter Weise sauer oder alkalisch durchgeführt werden.

Es wurde nun überraschenderweise gefunden, daß die erfindungsgemäß verwendete Gruppe der Verbindungen der Formel I und ihre physiologisch verträglichen Säureadditionssalze antiarrhythmische Wirkungen besitzen. Sie zeigen insbesondere Klasse III antiarrhythmische Eigenschaften und bewirken eine Verlängerung der effektiven Refraktärzeit im Herzen, welche zu einer Verlängerung des QT-Intervalls im EKG führt. Die Verbindungen besitzen ein günstiges Wirkprofil mit guter Verträglichkeit, langer Wirkungsdauer und einer so hohen Selektivität der antiarrhythmischen Wirkung gegenüber bradycarden und blutdrucksenkenden Eigenschaften, daß im antiarrhythmisch wirksamen Dosisbereich keine therapeutisch unerwünschte Beeinflußung der Herzfrequenz und/oder des Blutdruckes auftreten. Die Verbindungen zeichnen sich dadurch aus, daß die antiarrhythmische Wirkung unter tachycarden Bedingungen besonders gut ausgeprägt ist.

Die antiarrhythmischen Wirkungen der Verbindungen lassen sich in pharmakologischen Standardtestmethoden nachweisen. Beschreibung der pharmakologischen Untersuchungsmethoden.

### 1. Bestimmung der minimalen toxischen Dosis

Männlichen Mäusen von 20 bis 25 g Gewicht werden p.o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben. Die in Tabelle A angegebenen Beispielnummern beziehen sich auf die nachstehenden Herstellungsbeispiele.

**Tabelle A**

| Testsubstanz Beispiel Nr. | Minimale toxische Dosis mg/kg Maus p.o. |
|---|---|
| 1 | > 300 |
| 2 | > 300 |
| 6 | > 300 |
| 15 | > 300 |
| 20 | > 300 |
| 21 | > 300 |
| 22 | > 300 |
| 24 | > 300 |
| 47 | > 300 |

### 2. In vivo Untersuchung der antiarrhythmischen Eigenschaften der Substanzen unter tachycarden Bedingungen an narkotisierten Meerschweinchen.

Die Wirkungen der Substanzen auf die effektive Refraktärzeit (= ERP) und den Blutdruck bei i.v.-Applikation bei erhöhter Herzfrequenz wurden an narkotisierten Meerschweinchen untersucht. Den Tieren wurde unter Vollnarkose ein bipolarer Reizkatheter über eine Jugularvene in den rechten Ventrikel geschoben. Über diesen wurde die Herzfrequenz der Tiere durch elektrische Reizung während der gesamten Untersuchung auf ca. 150 % ihrer normalen Herzfrequenz gehalten. In die andere Jugularvene wurde eine Kanüle zur i.v.-Applikation der Testsubstanzen eingeführt. Während der Untersuchung wurden der systolische und der diastolische arterielle Blutdruck (= SAP und DAP) in einer Arteria carotis über einen Druckmesser (Statham-Drucktransducer) gemessen. Die Testsubstanzen wurden i.v. in steigenden Dosen (kumulativ) appliziert. Vor Applikation der ersten Dosis und jeweils 8 Minuten nach jeder Dosisgabe wurde die ERP mittels eines Doppelpulsprotokolls bestimmt. Die Dosis, bei der eine Verlängerung der ERP auf 115 % des Ausgangswertes erreicht wurde, wurde als effektive Dosis (= ERP-ED₁₁₅) berechnet. Als effektive Dosen für eine blutdrucksenkende Wirkung wurden die Dosis, bei der der SAP auf 85 % seines Ausgangswertes gesenkt wurde (= SAP-ED₈₅), und die Dosis, bei welcher der DAP auf 85 % seines Ausgangswertes gesenkt wurde (= DAP-ED₈₅), berechnet.

In der nachfolgenden Tabelle B werden die mit der vorstehend beschriebenen Methode erhaltenen Ergebnisse wiedergegeben. Die für die Testsubstanzen angegebenen Beispielsnummern beziehen sich auf die nachstehenden Herstellungsbeispiele.

**Tabelle B**

| Beispiel Nr. | antiarrhythmische Wirkung ERP-ED₁₁₅ in µmol/kg i.v. | Blutdrucksenkung ED₈₅ in µmol/kg i.v. | |
|---|---|---|---|
| | | DAP | SAP |
| 1 | 1 | 10 | 7 |
| 3 | 8 | > 32 | > 32 |
| 6 | 7 | > 10 | > 10 |
| 41 | 1 | > 10 | > 10 |
| 48 | 8 | - | - |

Die die Refraktärzeit verlängernde Wirkung der Substanzen läßt sich auch in in vitro Tests durch Bestimmung der funktionellen Refraktärzeit am isolierten Papillarmuskel der rechten Herzkammer von Meerschweinchen nachweisen.

Die vorstehenden Testergebnisse zeigen, daß die Verbindungen der Formel I antiarrhythmische Wirkungen besitzen und die effektive Refraktärzeit des Herzmuskels deutlich verlängern und daß eine effektive blutdrucksenkende Wirkung der Substanzen erst bei Dosen auftritt, die erheblich höher als die für die Refraktärzeitverlängerung effektiven Dosen sind.

Aufgrund ihres vorstehend beschriebenen Wirkungsprofils eignen sich die Substanzen zur Unterdrückung von tachycarden Herzrhythmusstörungen (Extrasystolen, Kammerflattern und - flimmern) und können zur Prophylaxe und Behandlung von Herzrhythmusstörungen in größeren Säugetieren und Menschen Verwendung finden. Insbesondere sind die Substanzen geeignet, das Auftreten von Tachyarrhythmien, d.h. Arrhythmien, die mit einem Anstieg der Herzfrequenz gekoppelt sind, zu verhindern.

Die zu verwendenden Dosen könnnen individuell verschieden sein und variieren naturgemäß je nach Art des zu behandelnden Zustandes, der verwendeten Substanz und der Applikationsform. Im allgemeinen eignen sich jedoch für Applikationen an größeren Säugetieren, insbesondere Menschen, Arzneiformen mit einem Wirkstoffgehalt von 0,5 bis 100, insbesondere 1 bis 25, mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester oder flüssiger Träger wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beipiele sollen die Erfindung näher erläutern, jedoch deren Umfang in keiner Weise beschränken.

### Beispiel 1:

### 7-(n-Butyl)-3-[(4-cyanophenyl)-sulfonyl]-9,9-dimethyl-3,7-diazabicyclo[3,3,1]nonan

Zu einer Lösung von 2,37 g 7-(n-Butyl)-9,9-dimethyl-3,7-diazabicyclo[3,3,1]nonan in 30 ml Dichlormethan wurde unter Eiskühlung eine Lösung von 2,5 g 4-Cyanobenzolsulfonsäurechlorid in 20 ml Dichlormethan zugetropft. Sodann wurde die Eiskühlung entfernt und das Reaktionsgemisch eine Stunde bei Raumtemperatur gerührt. Hierbei fiel das Hydrochlorid der Titelverbindung als weißer Niederschlag aus. Die Kristalle wurden abgesaugt und bei 60 °C in einem Vacuumtrockenschrank getrocknet. Es wurden 2,5 g 7-(n-Butyl)-3-[(4-cyanophenyl)-sulfonyl]-9,9-dimethyl-3,7-diazabicyclo[3,3,1]nonan-hydrochlorid mit einem Schmelzpunkt von 98 bis 99 °C erhalten.

### Beispiel 2:

### 7-(n-Butyl)-3-[(4-acetylaminophenyl)-sulfonyl]-9,9-dimethyl-3,7-diazabicyclo[3,3,1]nonan.

Zu einer Lösung von 39,3 g 7-(n-Butyl)-9,9-dimethyl-3,7-diazabicyclo[3,3,1]nonan und 29 ml Triethylamin in 700 ml Dichlormethan wurde unter Eiskühlung eine Lösung von 43,7 g 4-Acetamidosulfonsäurechlorid in 100 ml Dichlormethan zugetropft. Das Reaktionsgemisch wurde weitere 2 Stunden bei Raumtemperatur gerührt. Sodann wurde dem Reaktionsgemisch zur Aufarbeitung Wasser zugesetzt und das Gemisch zweimal mit Dichlormethan extrahiert. Die vereinigten Dichlormethanphasen wurden mit Magnesiumsulfat getrocknet und eingeengt. Als Rückstand wurden 42,4 g 7-(n-Butyl)-3-[(4-acetylaminophenyl)sulfonyl]-9,9-dimethyl-3,7-diazabicyclo[3,3,1]nonan erhalten.

2,9 g der Titelbase wurden in 20 ml Essigsäureethylester gelöst. Zu der Lösung wurde unter Eiskühlung eine Lösung von 1,1 g Weinsäure in 20 ml Aceton zugegeben. Hierbei fiel das Hydrogentartrat der Titelverbindung kristallin an. Die Kristalle wurden abgesaugt und im Vakuumtrockenschrank bei 50 °C getrocknet. Es wurden 3,6 g Monohydrogentartrat der Titelverbindung mit einem Schmelzpunkt von 130 °C erhalten.

### Beispiel 3:

### 7-(n-Butyl)-3-[(4-(imidazol-1-yl)-phenyl)-sulfonyl]-9,9-dimethyl-3,7-diazabicyclo[3,3,1]nonan.

A) Zu einer Lösung von 7,8 g 7-(n-Butyl)-9,9-dimethyl-3,7-diazabicyclo[3,3,1]nonan in 40 ml Dichlormethan wurde unter Eiskühlung eine Lösung von 7,2 g 4-Fluorbenzuolsulfonsäurechlorid in 10 ml Dichlormethan zugetropft. Sodann wurde die Eiskühlung entfernt und das Reaktionsgemisch 1 Stunde bei Raumtemperatur gerührt. Zur Aufarbeitung wurde dem Reaktionsgemisch wäßrige Natriumbicarbonatlösung zugesetzt und zweimal mit Dichlormethan extrahiert. Die vereinigten Dichlormethanextrakte wurden mit Magnesiumsulfat getrocknet und eingeengt. Es wurden 12,9 g 7-(n-Butyl)-3-[(4-fluorphenyl)-sulfonyl]-9,9-dimethyl-3,7-diazabicy-clo[3,3,1]nonan erhalten.
B) 12,9 g des vorstehend erhaltenen Produktes, 5,8 g Kaliumcarbonat und 2,4 g Imidazol wurden in 200 ml Dimethylsulfoxid 10 Stunden auf 120 °C erhitzt. Anschließend wurde das Reaktionsgemisch abgekühlt, das Kaliumcarbonat abfiltriert und das Filtrat eingeengt. Der verbleibende Rückstand wurde mit wäßriger Natriumhydroxidlösung versetzt und zweimal mit Dichlormethan extrahiert. Die vereinigten Dichlormethanextrakte wurden mit Magnesiumsulfat getrocknet und eingeengt. Die als öliger Rückstand verbleibende rohe Titelverbindung wurde durch Chromatographie über Tonerde unter Verwendung von Essigsäureäthylester als Elutionsmittel gereinigt. Es wurden 4,2 g 7-(n-Butyl)-3-[(4-(imidazol-1-yl)-phenyl)-sulfonyl]-9,9-dimethyl-3,7-diazabicyclo[3,3,1]nonan mit einem Schmelzpunkt von 148 bis 150 °C erhalten.

### Beispiel 4:

### 7-(n-Butyl)-3-[(4-(4-cyanobenzoylamino)-phenyl)-sulfonyl]-9,9-dimethyl-3,7-diazabicyclo[3,3,1]nonan

A) 5,1 g 7-(n-Butyl)-3-[(4-acetylaminophenyl)-sulfonyl]-9,9-dimethyl-3,7-diazabicyclo[3,3,1]nonan (Herstellung siehe Beispiel 2) und 1,5 g Kaliumhydroxid wurden in 100 ml Ethanol 6 Stunden am Rückfluß erhitzt. Sodann wurde das Reaktionsgemisch abgekühlt, Wasser zugegeben, und zweimal mit Diethylether extrahiert. Die vereinigten Etherextrakte wurden mit Magnesiumsulfat getrocknet und eingeengt. Es wurden 4,0 g 7-(n-Butyl)-3-[(4-aminophenyl)-sulfonyl]-9,9-dimethyl-3,7-diazabicyclo[3,3,1]nonan erhalten, welche ohne weitere Reinigung in der nachfolgenden Reaktionsstufe eingesetzt wurden.
B) 1,4 g des vorstehend erhaltenen Produktes und 0,63 g 4-Cyanobenzoylchlorid wurden in 20 ml Dichlormethan gelöst. Das Reaktionsgemisch wurde 1 Stunde bei Raumtemperatur reagieren gelassen. Zur Aufarbeitung wurde das Reaktionsgemisch durch Zugabe von wäßriger Natriumhydroxidlösung alkalisch gestellt. Sodann wurde die wäßrige Phase abgetrennt und zweimal mit Dichlormethan extrahiert. Die vereinigten Dichlormethanphasen wurden mit Magnesiumsulfat getrocknet und eingeengt. Die als kristalliner Rückstand erhaltene rohe Titelverbindung wurde aus Diethyläther umkristallisiert, und die erhaltenen Kristalle wurden bei 60 °C in einem Vacuumtrockenschrank getrocknet. Es wurden 1,3 g 7-(n-Butyl)-3-[(4-(4-cyanobenzoylamino)-phenyl)-sulfonyl]-9,9-dimethyl-3,7-diazabicyclo-[3,3,1]nonan mit einem Schmelzpunkt von 145 °C erhalten.

Nach den in den vorstehenden Beispielen beschriebenen Verfahren können auch die in der folgenden Tabelle angeführten Verbindungen der Formel I hergestellt werden.

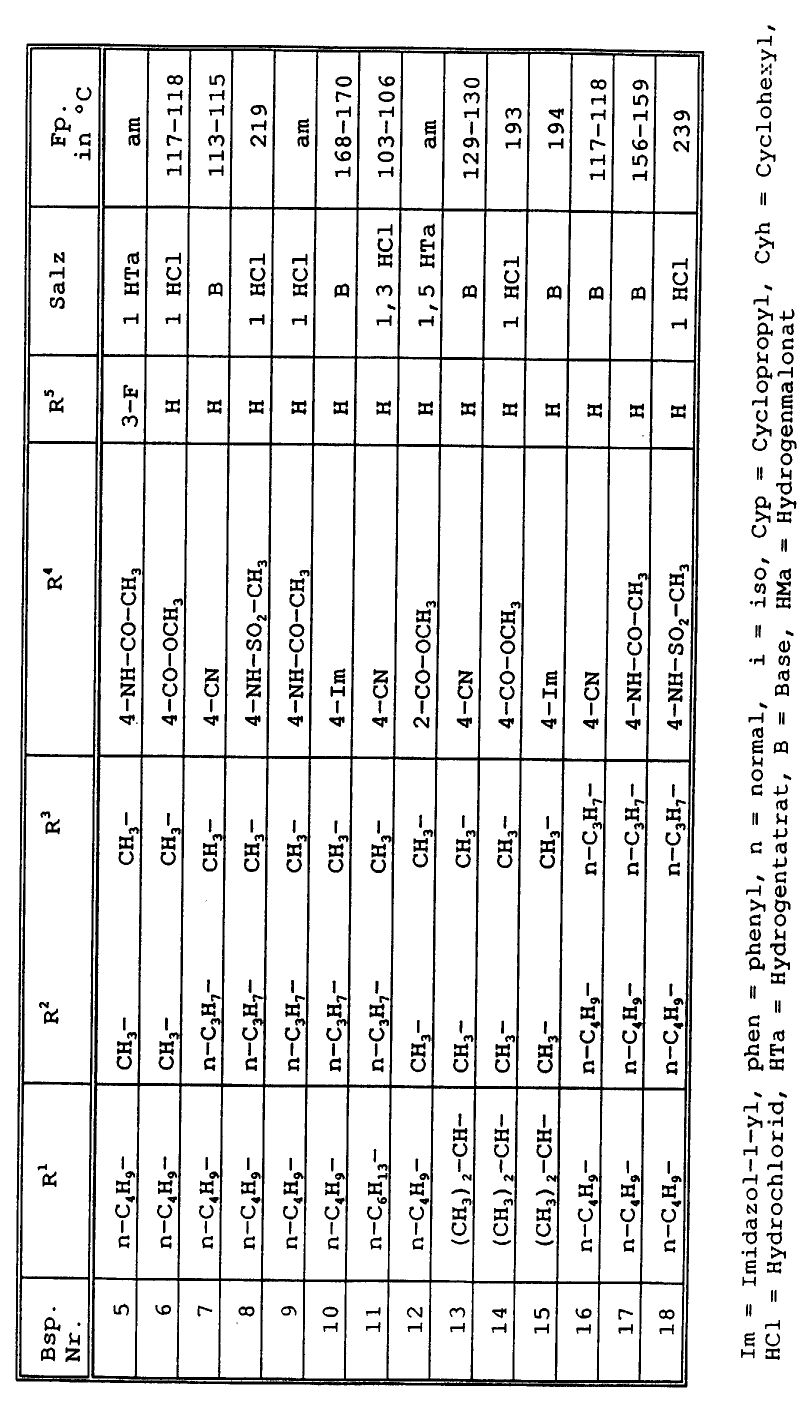

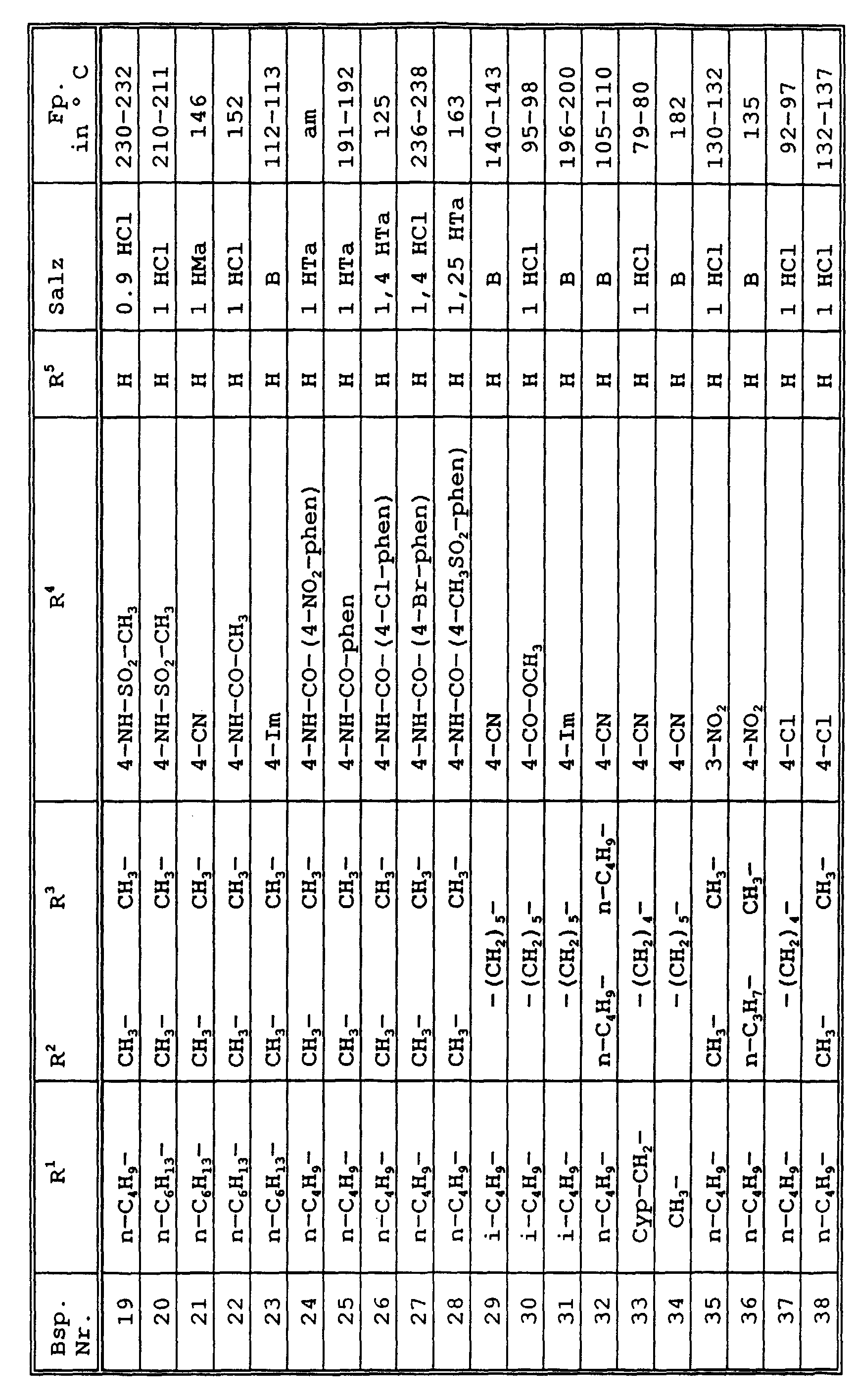

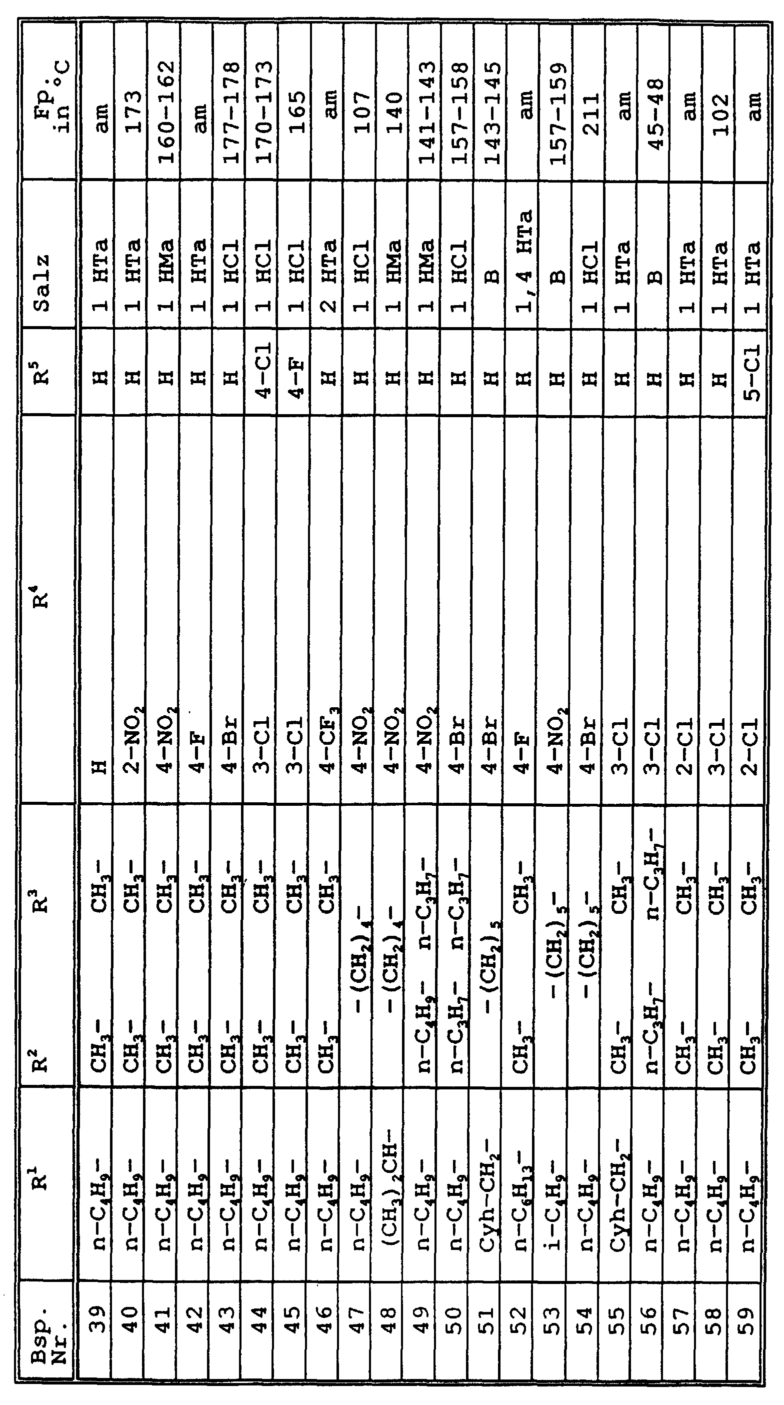

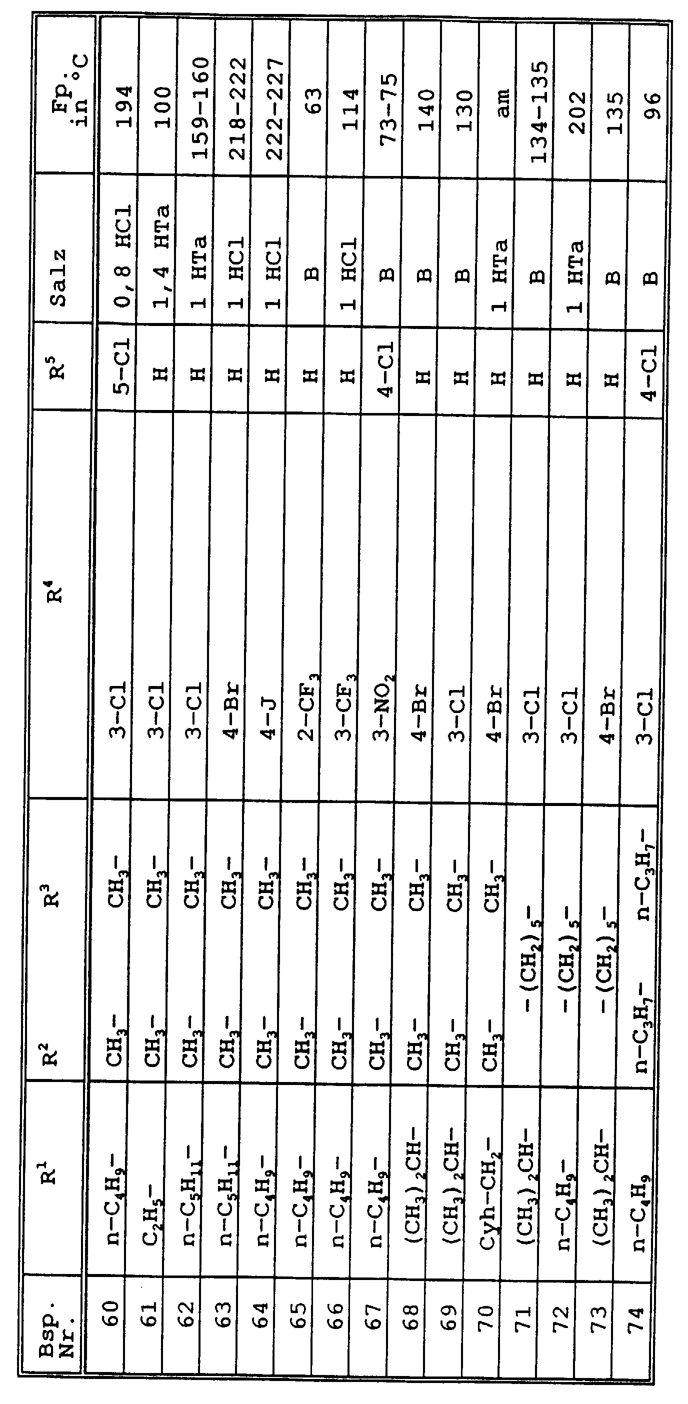

### Beispiel I:

### 7-(n-Butyl)-3-[(4-cyanophenyl)-sulfonyl]-9,9-dimethyl-3,7-diazabicyclo[3,3,1]nonan-hydrochlorid enthaltende Tabletten.

Man stellte Tabletten in folgender Zusammensetzung pro Tablette her:

| | |
|---|---|
| 7-(n-Butyl)-3-[(4-cyanophenyl)-sulfonyl]-9,9-dimethyl-3,7-diazabicyclo[3,3,1]nonan-hydrochlorid | 20 mg |
| Maisstärke | 60 mg |
| Milchzucker | 135 mg |
| Gelatine (als 10 %-ige Lösung) | 6 mg |

Der Wirkstoff, die Maisstärke und der Milchzucker wurden mit der 10 %-igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

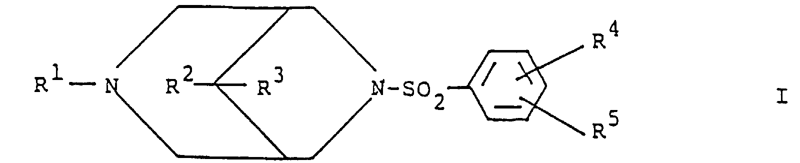

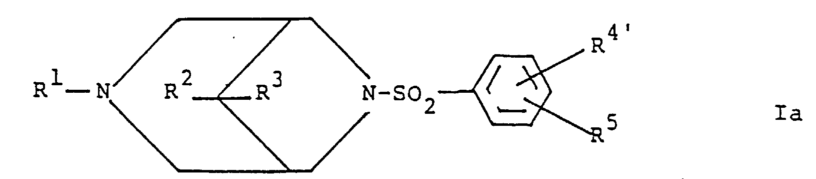

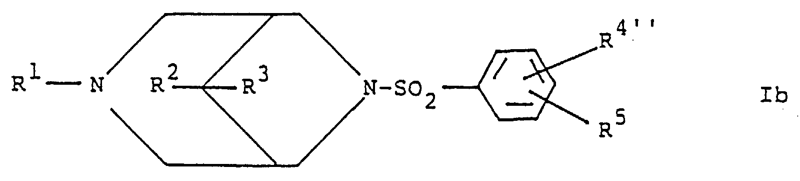

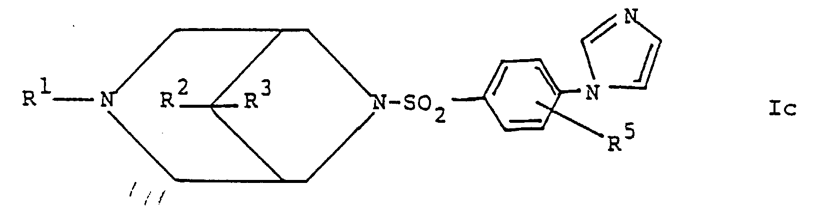

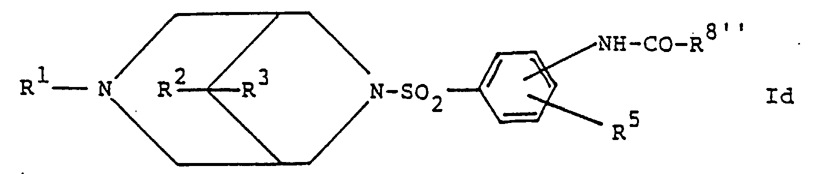

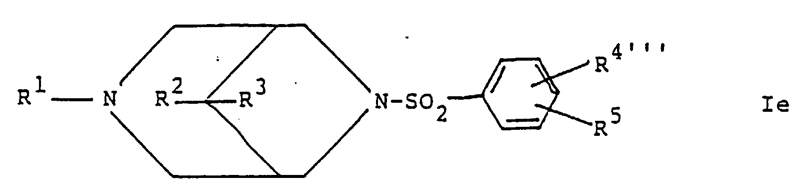

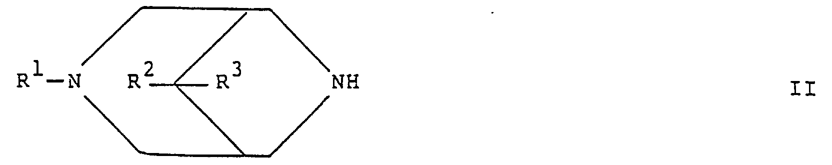

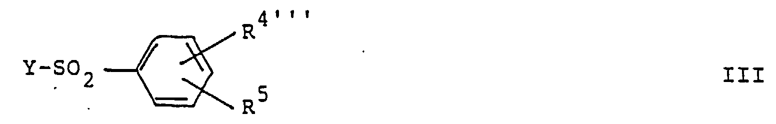

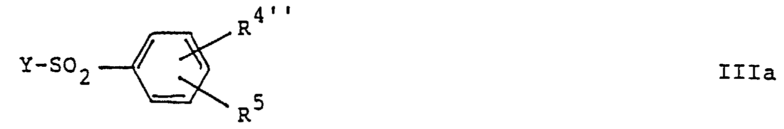

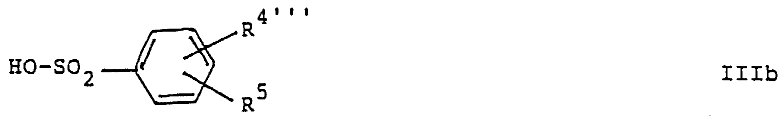

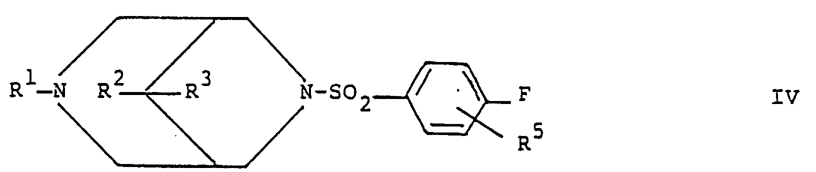

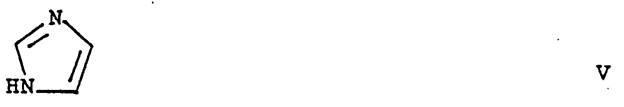

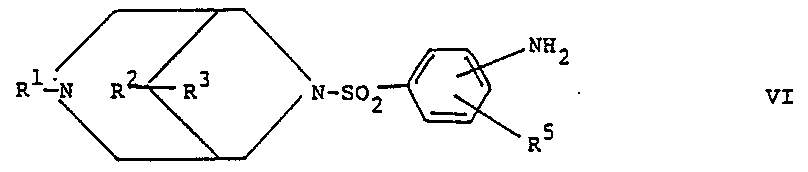

X-CO-R^{8"} VII

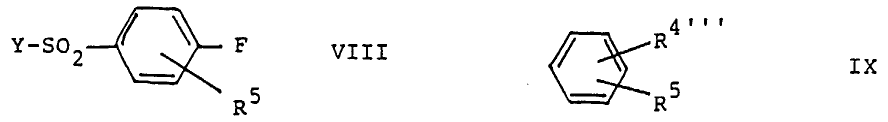

## Patentansprüche

1. Verwendung von 3-Phenylsulfonyl-3,7-diazabicyclo-[3,3,1]nonan-Verbindungen der allgemeinen Formel I 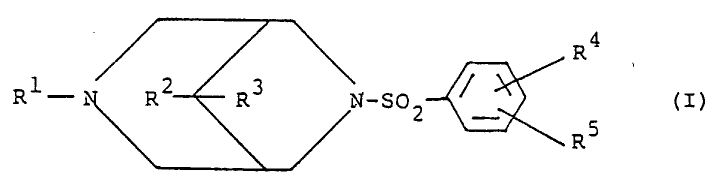 worin
R¹ eine Alkylgruppe mit 1-6 Kohlenstoffatomen oder eine Cycloalkylalkylgruppe mit 4-7 Kohlenstoffatomen bedeutet,
R² C₁-C₄ Alkyl bedeutet und
R³ C₁-C₄ Alkyl bedeutet oder
R² und R³ gemeinsam eine Alkylenkette mit 3-6 Kohlenstoffatomen bilden,
R⁴ für Halogen, Nitro, Trifluormethyl oder Cyano, für eine R⁶-O-CO-Gruppe, worin R⁶ C₁-C₄ Alkyl bedeutet, für eine R⁷-SO₂-NH-Gruppe, worin R⁷ C₁-C₄ Alkyl bedeutet, oder für eine R⁸-CO-NH-Gruppe, worin R⁸ C₁-C₄ Alkyl oder eine Phenylgruppe, welche gegebenenfalls durch Halogen, Cyano, Nitro oder einen R⁹-SO₂-Rest, worin R⁹ C₁-C₄ Alkyl ist, substituiert ist, bedeutet, oder für einen in 4-Stellung des Phenylringes angeordneten Imidazolylrest steht, und
R⁵ Wasserstoff oder Halogen bedeutet, und
deren physiologisch verträglichen Säureadditionssalzen zur Herstellung von pharmazeutischen Zubereitungen zur Behandlung von Herzrhythmusstörungen in größeren Säugetieren und Menschen.

2. Verwendung gemäß Anspruch 1, dadurch gekennzeichnet, daß Verbindungen eingesetzt werden, worin R¹ eine Alkylgruppe mit 3-5 Kohlenstoffatomen bedeutet und R² und R³ je C₁-C₄ Alkyl bedeuten.

3. Verwendung gemäß einem der vorstehenden Ansprüche, dadurch gekennzeichnet, daß Verbindungen eingesetzt werden, worin R⁴ für Cyano oder für einen in 4-Stellung des Phenylringes angeordneten Imidazolylrest steht.

4. Antiarrhythmisch wirksame Arzneimittel, dadurch gekennzeichnet, daß sie eine antiarrhythmisch wirksame Menge einer Verbindung gemäß Anspruch 1 und übliche pharmazeutische Hilfs- und/oder Trägerstoffe enthalten.

5. Verbindungen der allgemeinen Formel Ia 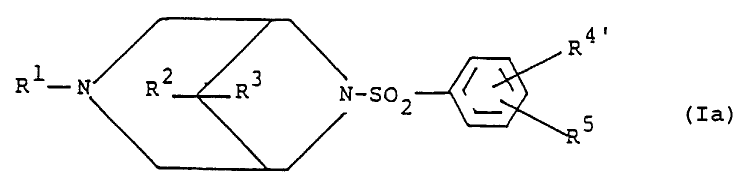 worin
R¹ eine Alkylgruppe mit 1-6 Kohlenstoffatomen oder eine Cycloalkylalkylgruppe mit 4-7 Kohlenstoffatomen bedeutet,
R² C₁-C₄ Alkyl bedeutet und
R³ C₁-C₄ Alkyl bedeutet oder
R² und R³ gemeinsam eine Alkylenkette mit 3-6 Kohlenstoffatomen bilden,
R^{4'} für Cyano, für eine R⁶-O-CO-Gruppe, worin R⁶ C₁-C₄ Alkyl bedeutet, für eine R⁷-SO₂-NH-Gruppe, worin R⁷ C₁-C₄ Alkyl bedeutet, oder für eine R⁸-CO-NH-Gruppe, worin R⁸ C₁-C₄ Alkyl oder eine Phenylgruppe, welche gegebenenfalls durch Halogen, Cyano, Nitro oder einen R⁹-SO₂- Rest, worin R⁹ C₁-C₄ Alkyl ist, substituiert ist, bedeutet, oder für einen in 4-Stellung des Phenylringes angeordneten Imidazolylrest steht, und
R⁵ Wasserstoff oder Halogen bedeutet, und
deren physiologisch verträgliche Säureadditionssalze.

6. Verfahren zur Herstellung von Verbindungen der allgemeinen Formel Ia 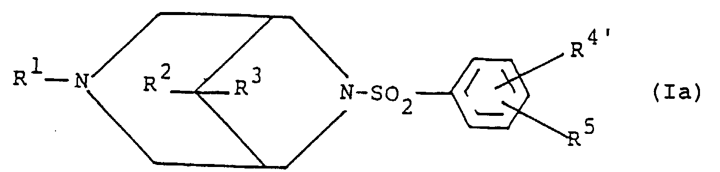 worin
R¹ eine Alkylgruppe mit 1-6 Kohlenstoffatomen oder eine Cycloalkylalkylgruppe mit 4-7 Kohlenstoffatomen bedeutet,
R² C₁-C₄ Alkyl bedeutet und
R³ C₁-C₄ Alkyl bedeutet oder
R² und R³ gemeinsam eine Alkylenkette mit 3-6 Kohlenstoffatomen bilden,
R^{4'} für Cyano, für eine R⁶-O-CO-Gruppe, worin R⁶ C₁-C₄ Alkyl bedeutet, für eine R⁷-SO₂-NH-Gruppe, worin R⁷ C₁-C₄ Alkyl bedeutet, oder für eine R⁸-CO-NH-Gruppe, worin R⁸ C₁-C₄ Alkyl oder eine Phenylgruppe, welche gegebenenfalls durch Halogen, Cyano, Nitro oder einen R⁹-SO₂- Rest, worin R⁹ C₁-C₄ Alkyl ist, substituiert ist, bedeutet, oder für einen in 4-Stellung des Phenylringes angeordneten Imidazolylrest steht, und
R⁵ Wasserstoff oder Halogen bedeutet, und
deren physiologisch verträglichen Säureadditionssalzen dadurch gekennzeichnet, daß man
a) zur Herstellung von Verbindungen der allgemeinen Formel Ib 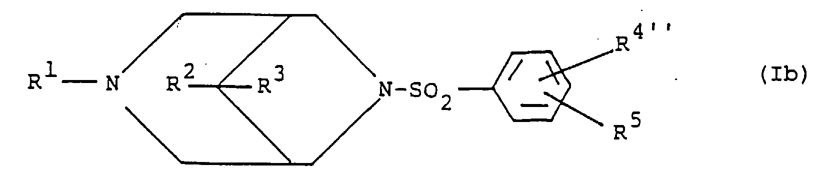 worin R¹, R², R³ und R⁵ obige Bedeutung besitzen und R^{4"} für Cyano, für eine R⁶-O-CO-Gruppe, worin R⁶ obige Bedeutung besitzt, für eine R⁷-SO₂-NH-Gruppe, worin R⁷ obige Bedeutung besitzt, oder für eine R^{8'}-CO-NH-Gruppe, worin R^{8'} C₁-C₄ Alkyl bedeutet, steht
Verbindungen der allgemeinen Formel II 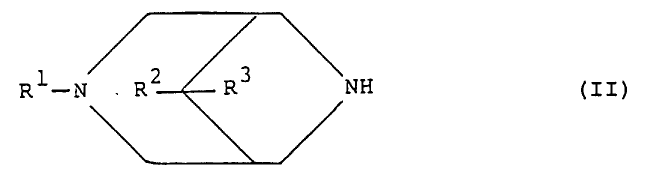 worin R¹, R² und R³ obige Bedeutung besitzen, mit Verbindungen der allgemeinen Formel IIIa 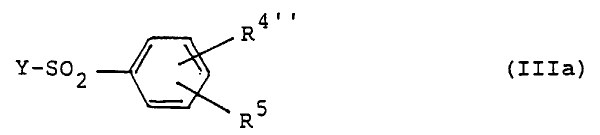 worin R^{4"} und R⁵ obige Bedeutung besitzen und Y für eine reaktive Gruppe steht, umsetzt, oder
b) zur Herstellung von Verbindungen der allgemeinen Formel Ic 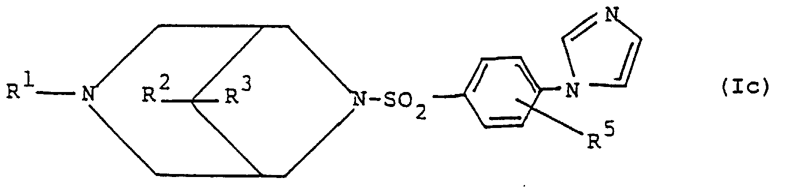 worin R¹, R², R³ und R⁵ obige Bedeutung besitzen, Verbindungen der allgemeinen Formel IV 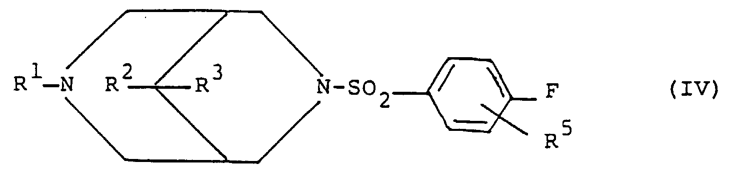 worin R¹, R², R³ und R⁵ obige Bedeutung besitzen, mit Imidazol der Formel V 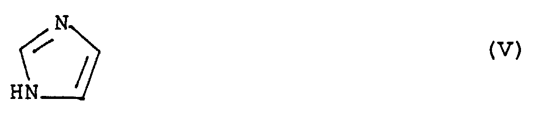 umsetzt, oder
c) zur Herstellung von Verbindungen der allgemeinen Formel Id 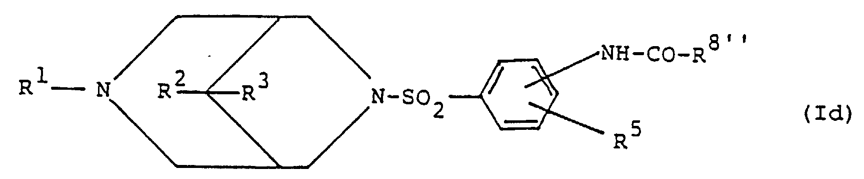 worin R¹, R², R³ und R⁵ obige Bedeutung besitzen und R^{8''} eine Phenylgruppe, welche gegebenenfalls durch Halogen, Cyano, Nitro oder einen R⁹-SO₂-Rest, worin R⁹ C₁-C₄ Alkyl ist, substituiert ist, bedeutet, Verbindungen der allgemeinen Formel VI 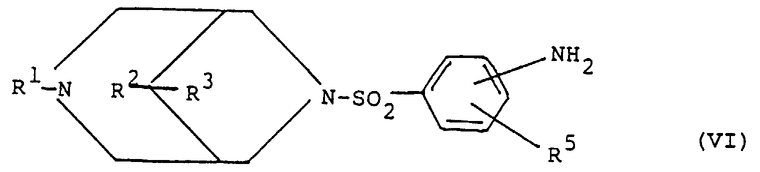 worin R¹, R², R³ und R⁵ obige Bedeutung besitzen, mit Säuren oder reaktionsfähigen Säurederivaten der allgemeinen Formel VII
X-CO-R^{8''} (VII)
worin R^{8''} obige Bedeutung besitzt und X Hydroxy oder eine reaktive Gruppe bedeutet, acyliert,
und gegebenenfalls freie Verbindungen der Formel Ia in ihre Säureadditionssalze überführt oder die Säureadditionssalze in die freien Verbindungen der Formel Ia überführt.

## Claims

1. Use of 3-phenylsulphonyl-3,7-diazabicyclo-[3.3.1]nonane compounds of the general formula I 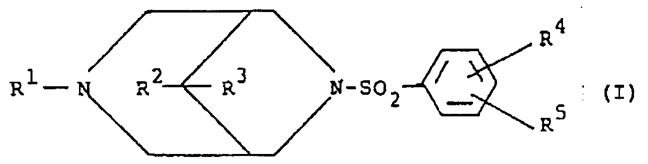 in which
R¹ is an alkyl group having 1-6 carbon atoms or a cycloalkylalkyl group having 4-7 carbon atoms,
R² is C₁-C₄ alkyl and
R³ is C₁-C₄ alkyl or
R² and R³ together form an alkylene chain having 3-6 carbon atoms,
R⁴ is halogen, nitro, trifluoromethyl or cyano, an R⁶-O-CO- group in which R⁶ is C₁-C₄ alkyl, an R⁷-SO₂-NH- group in which R⁷ is C₁-C₄ alkyl, or an R⁸-CO-NH- group in which R⁸ is C₁-C₄ alkyl or a phenyl group which is optionally substituted by halogen, cyano, nitro or an R⁹-SO₂- radical in which R⁹ is C₁-C₄ alkyl, or an imidazolyl radical located in position 4 of the phenyl ring, and
R⁵ is hydrogen or halogen, and
their physiologically tolerable acid addition salts for the production of pharmaceutical preparations for the treatment of cardiac arrhythmias in larger mammals and humans.

2. Use according to Claim 1, characterized in that compounds are used in which R¹ is an alkyl group having 3-5 carbon atoms and R² and R³ are each C₁-C₄ alkyl.

3. Use according to one of the above claims, characterized in that compounds are used in which R⁴ is cyano or an imidazolyl radical located in position 4 of the phenyl ring.

4. Antiarrhythmically effective medicaments, characterized in that they contain an antiarrhythmically effective amount of a compound according to Claim 1 and customary pharmaceutical auxiliaries and/or excipients.

5. Compounds of the general formula Ia 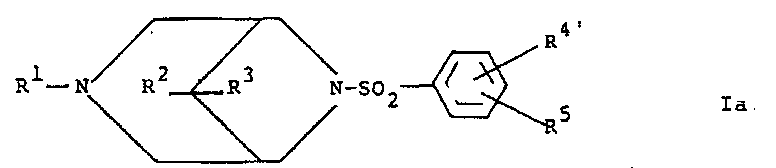 in which
R¹ is an alkyl group having 1-6 carbon atoms or a cycloalkylalkyl group having 4-7 carbon atoms,
R² is C₁-C₄ alkyl and
R³ is C₁-C₄ alkyl or
R² and R³ together form an alkylene chain having 3-6 carbon atoms,
R^{4'} is cyano, an R⁶-O-CO- group in which R⁶ is C₁-C₄ alkyl, an R⁷-SO₂-NH- group in which R⁷ is C₁-C₄ alkyl, or an R⁸-CO-NH- group in which R⁸ is C₁-C₄ alkyl or a phenyl group which is optionally substituted by halogen, cyano, nitro or an R⁹-SO₂- radical in which R⁹ is lower alkyl, or an imidazolyl radical located in position 4 of the phenyl ring, and
R⁵ is hydrogen or halogen, and
their physiologically tolerable acid addition salts.

6. Process for the preparation of compounds of the general formula Ia 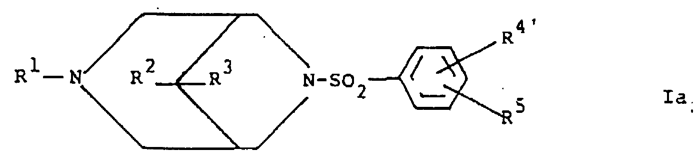 in which
R¹ is an alkyl group having 1-6 carbon atoms or a cycloalkylalkyl group having 4-7 carbon atoms,
R² is C₁-C₄ alkyl and
R³ is C₁-C₄ alkyl or
R² and R³ together form an alkylene chain having 3-6 carbon atoms,
R^{4'} is cyano, an R⁶-O-CO- group in which R⁶ is C₁-C₄ alkyl, an R⁷-SO₂-NH- group in which R⁷ is C₁-C₄ alkyl, or an R⁸-CO-NH- group in which R⁸ is C₁-C₄ alkyl or a phenyl group which is optionally substituted by halogen, cyano, nitro or an R⁹-SO₂- radical in which R⁹ is C₁-C₄ alkyl, or an imidazolyl radical located in position 4 of the phenyl ring, and
R⁵ is hydrogen or halogen, and
their physiologically tolerable acid addition salts, characterized in that
a) for the preparation of compounds of the general formula Ib 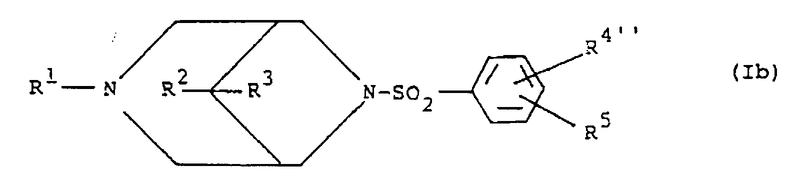 in which R¹, R², R³ and R⁵ have the above meaning and R^{4"} is cyano, an R⁶-O-CO- group in which R⁶ has the above meaning, an R⁷-SO₂-NH- group in which R⁷ has the above meaning, or an R^{8'}-CO-NH- group in which R^{8'} is C₁-C₄ alkyl,
compounds of the general formula II 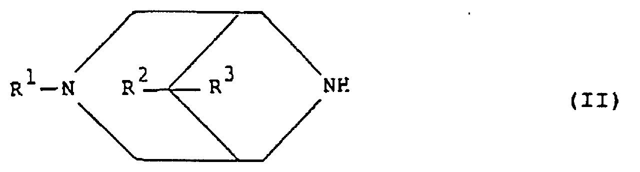 in which R¹, R² and R³ have the above meaning, are reacted with compounds of the general formula IIIa 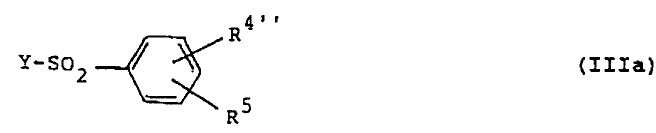 in which R^{4"} and R⁵ have the above meaning and Y is a reactive group, or
b) for the preparation of compounds of the general formula Ic 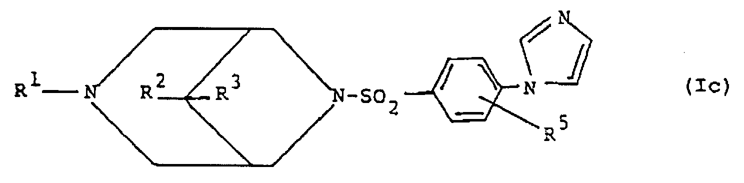 in which R¹, R², R³ and R⁵ have the above meaning,
compounds of the general formula IV 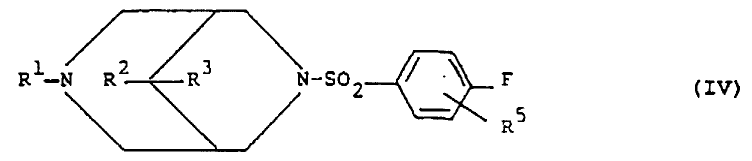 in which R¹, R², R³ and R⁵ have the above meaning, are reacted with imidazole of the formula V 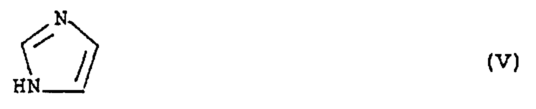 or
c) for the preparation of compounds of the general formula Id 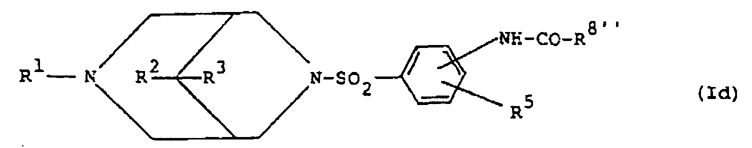 in which R¹, R², R³ and R⁵ have the above meaning and R^{8"} is a phenyl group which is optionally substituted by halogen, cyano, nitro or an R⁹-SO₂- radical in which R⁹ is C₁-C₄ alkyl, compounds of the general formula VI 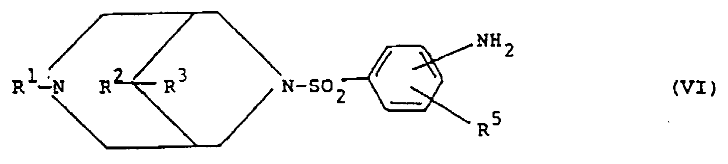 in which R¹, R², R³ and R⁵ have the above meaning, are acylated using acids or reactive acid derivatives of the general formula VII
X-CO-R^{8"} (VII)
in which R^{8"} has the above meaning and X is hydroxyl or a reactive group,
and free compounds of the formula Ia are optionally converted into their acid addition salts or the acid addition salts are converted into the free compounds of the formula Ia.

## Revendications

1. Utilisation de 3-phénylsulfonyl-3,7-diazabicyclo[3,3,1]-nonanes, composés de formule générale I 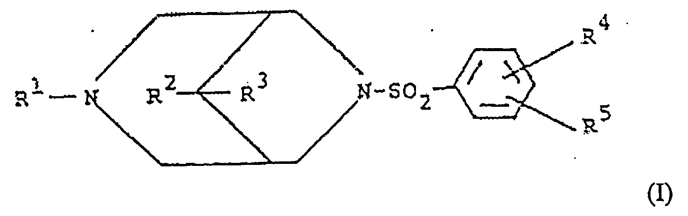 dans laquelle
R¹ représente un groupe alkyle ayant 1 à 6 atomes de carbone ou un groupe cycloalkyl alkyle ayant 4 à 7 atomes de carbone;
R² représente un groupe alkyle en C₁-C₄ , et
R³ représente un groupe alkyle en C₁-C₄ ou bien,
R² et R³ forment ensemble une chaîne alkylène ayant 3 à 6 atomes de carbone,
R⁴ représente un atome d'halogène, un groupe nitro, trifluorométhyle ou cyano, un groupe R⁶-O-CO- (dans lequel R⁶ représente un groupe alkyle en C₁-C₄), un groupe R⁷-SO₂-NH- (dans lequel R⁷ représente R⁷ représente un groupe alkyle en C₁-C₄ ) ou un groupe R⁸-CO-NH- (dans lequel R⁸ représente un groupe alkyle en C₁-C₄ ou un groupe phényle, qui est éventuellement substitué par de l'halogène, par un reste cyano, nitro ou R⁹-SO₂ dans lequel R⁹ représente un groupe alkyle en C₁-C₄ ) ou R⁹ représente un reste imidazolyle fixé en position 4 du noyau phényle , et
R⁵ représente un atome d'hydrogène ou d'halogène)
et leurs sels d'addition d'acides physiologiquement tolérables, pour produire des préparations pharmaceutiques destinées à traiter des troubles du rythme cardiaque chez des grands mammifères et des êtres humains.

2. Utilisation selon la revendication 1, caractérisée en ce qu'on utilise des composés dans lesquels R¹ représente un groupe alkyle ayant 3 à 5 atomes de carbone, R² et R³ représentent chacun un groupe alkyle en C₁-C₄.

3. Utilisation selon l'une des revendications précédentes, caractérisée en ce qu'on utilise des composés dans lesquels R⁴ représente un groupe cyano ou un reste imidazolyle fixé en position 4 du noyau phényle.

4. Médicaments à action anti-arythmique, caractérisés en ce qu'ils contiennent une quantité, active du point de vue anti-arythmique, d'un composé selon la revendication 1 et des adjuvants et des véhicules ou excipients, ou bien des adjuvants et/ou des véhicules ou excipients pharmaceutiques usuels.

5. Composés de formule générale Ia : 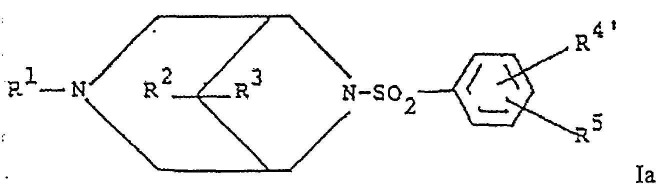 [dans laquelle
R¹ représente un groupe alkyle ayant 1 à 6 atomes de carbone ou un groupe cycloalkyl alkyle ayant 4 à 7 atomes de carbone;
R² représente un groupe alkyle en C₁-C₄, et
R³ représente un groupe alkyle en C₁-C₄, ou bien
R² et R³ forment ensemble une chaîne alkylène ayant 3 à 6 atomes de carbone,
R⁴' représente un groupe cyano, un groupe R⁶-O-CO- (dans lequel R⁶ représente un groupe alkyle en C₁-C₄), un groupe R⁷-SO₂-NH- (dans lequel R⁷ représente un groupe alkyle en C₁-C₄) ou un groupe R⁸-CO-NH- dans lequel R⁸ représente un groupe alkyle en C₁-C₄ ou un groupe phényle qui est éventuellement substitué par un atome d'halogène, par un reste cyano, nitro ou R⁹-SO₂- (dans lequel R⁹ représente un groupe alkyle en C₁-C₄),ou bien un reste imidazolyle fixé en position 4 du noyau phényle, et
R⁵ représente un atome d'hydrogène ou d'halogène) et leurs sels d'addition d'acides physiologiquement tolérables.

6. Procédé de production de composés de formule générale Ia : 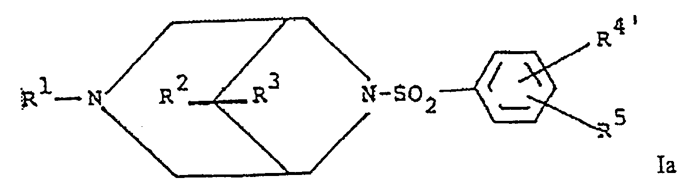 dans laquelle
R¹ représente un groupe alkyle ayant 1 à 6 atomes de carbone ou un groupe cycloalkyl alkyle ayant 4 à 7 atomes de carbone;
R² représente un groupe alkyle en C₁-C₄, et
R³ représente un groupe alkyle en C₁-C₄ , ou bien
R² et R³ forment ensemble une chaîne alkylène ayant 3 à 6 atomes de carbone,
R⁴' représente un groupe cyano, un groupe R⁶-O-CO- (dans lequel R⁶ représente un groupe alkyle en C₁-C₄ ), un groupe R⁷-SO₂-NH- (dans lequel R⁷ représente un groupe alkyle en C₁-C₄) ou un groupe R⁸-CO-NH- dans lequel R⁸ représente un groupe alkyle en C₁-C₄ ou un groupe phényle qui est éventuellement substitué par de l'halogène, par un reste cyano, nitro ou R⁹-SO₂- (dans lequel R⁹ représente un groupe alkyle en C₁-C₄),ou bien R⁴' représente un reste imidazolyle fixé en position 4 du noyau phényle, et
R⁵ représente un atome d'hydrogène ou d'halogène
et de leurs sels d'addition d'acides physiologiquement tolérables, procédé caractérisé en ce que :
a) pour produire des composés de formule (Ib) : 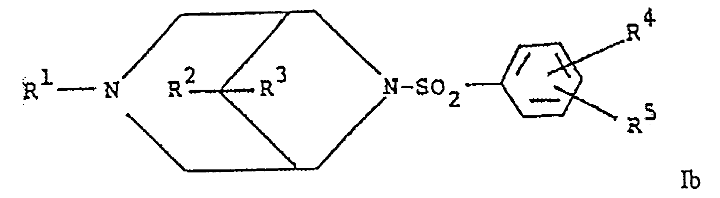 dans laquelle R¹, R², R³ et R⁵ ont le sens précité, et R⁴" représente un groupe cyano, un groupe R⁶-O-CO- dans lequel R⁶ a le sens précité), un groupe R⁷-SO₂-NH- (dans lequel R⁷ a le sens précité ou un groupe R⁸'-CO-NH- (dans lequel R⁸' représente un groupe alkyle en C₁-C₄), on fait réagir des composés de formule générale II 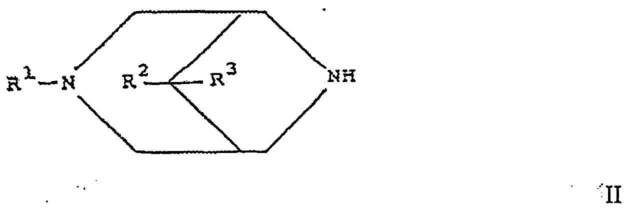 dans laquelle R¹, R², R³ et R⁵ ont le sens précité) avec des composés de formule III a 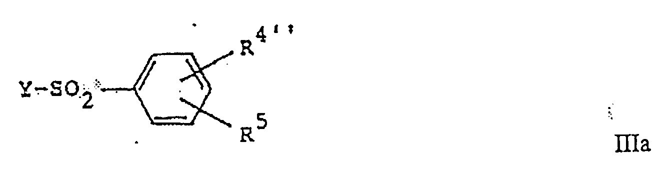 dans laquelle R^{4"} et R⁵ ont le sens ci-dessus; et Y représente un groupe réactif , ou bien
b) pour produire des composés de formule Ic 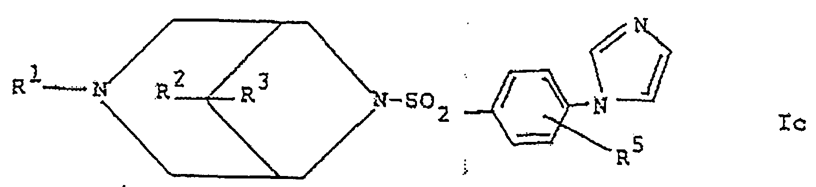 dans laquelle R¹, R², R³ et R⁵ ont le sens précité, on fait réagir des composés de formule (IV) 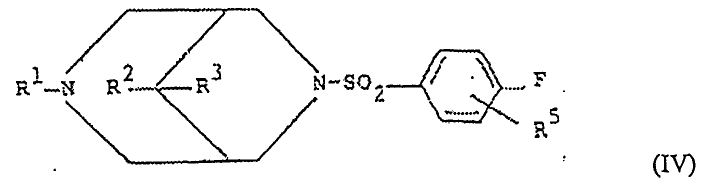 dans laquelle R¹, R², R³ et R⁵ ont le sens ci-dessus avec l'imidazole de formule (V) 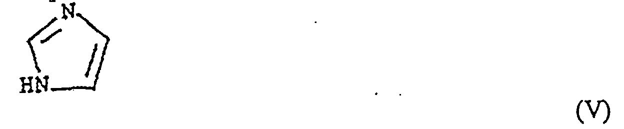 ou bien,
c) pour produire des composés de composés de formule (Id) 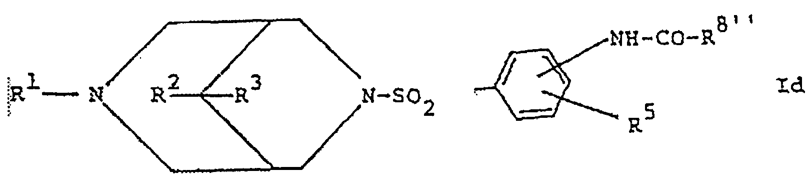 dans laquelle R¹, R², R³ et R⁵ ont le sens ci-dessus, et R⁸" représente un groupe phényle, qui est éventuellement substitué par de l'halogène, par un reste cyano, nitro ou R⁹-SO₂- , dans lequel R⁹ représente un groupe alkyle en C₁-C₄ , on acyle des composés de formule (VI) 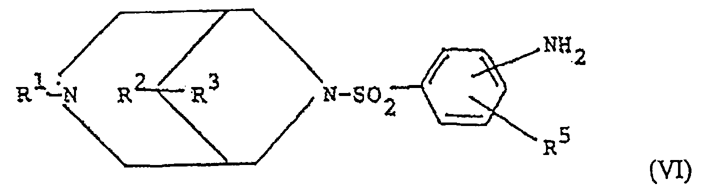 dans laquelle R¹, R², R³ et R⁵ ont le sens ci-dessus, avec des acides ou des dérivés réactifs d'acides de formule générale (VII)
X-CO-R^{8"}
dans laquelle R⁸" a le sens ci-dessus et X représente un groupe hydroxyle ou un groupe réactif),
et éventuellement, on transforme des composés libres, de formule (Ia), en leurs sels d'addition d'acides ou bien on transforme les sens d'addition d'acides en composés libres de formule (Ia).
